# EUROPEAN PATENT APPLICATION

(11) **EP 2 082 770 A1**
(43) Date of publication of application: **29.07.2009**
(21) Application number: 08100889.8
(22) Date of filing: 24.01.2008
(51) Int. Cl.: A61M 15/00

(54) **Inhaler**

(71) Applicant: Vectura Delivery Devices Limited, Chippenham, Wiltshire SN14 6FH (GB)
(72) Inventor: Eason, Stephen, Norfolk IP22 1RX (GB); Sarkar, Matthew, Cambridge CB4 3JU (GB); Tyres, Ben, Lancashire LA6 2ET (GB); Cameron, Jamie, Inverness Shire, PH33 6LL (GB); Banister, Stuart, Peterborough PE2 8TN (GB); Sheldon, Mike, Cambridge CB3 0HN (GB)
(74) Representative: Grey, Ian Michael

(57) **Abstract**

An inhaler (10) comprising a housing (11) to receive a strip (14) having a plurality of blisters (14a), each blister having a puncturable lid (14c) and containing a dose of medicament for inhalation by a user, a mouthpiece (13) through which a dose of medicament is inhaled by a user, a cap (12) to cover the mouthpiece and, a blister piercing element (16) to pierce the lid of a blister is disclosed. The cap is rotatable to drive the strip to sequentially move each blister into alignment with the blister piercing element and an actuator (18) is operable in response to rotation of the cap to cause the blister piercing element to puncture the lid of an aligned blister such that, when a user inhales through the mouthpiece, an airflow through the blister is generated to entrain the dose contained therein and carry it out of the blister and via the mouthpiece into the user's airway.

## Description

The present invention relates to an inhalation device for oral or nasal delivery of medicament in powdered form. More specifically, the invention relates to an inhaler having a housing to receive a strip having a plurality of blisters spaced along the length of the strip, each blister having a puncturable lid and containing a dose of medicament for inhalation by a user. The invention also relates to an inhaler containing a strip of blisters each having a puncturable lid and containing a dose of medicament for inhalation by a user of the device according to the invention.

Oral or nasal delivery of a medicament using an inhalation device is a particularly attractive method of drug administration as these devices are relatively easy for a patient to use discreetly and in public. As well as delivering medicament to treat local diseases of the airway and other respiratory problems, they have more recently also been used to deliver drugs to the bloodstream via the lungs, thereby avoiding the need for hypodermic injections.

It is common for dry powder formulations to be pre-packaged in individual doses, usually in the form of capsules or blisters which each contain a single dose of the powder which has been accurately and consistently measured. A blister is generally cold formed from a ductile foil laminate or a plastics material and includes a puncturable lid which is permanently heat-sealed around the periphery of the blister during manufacture and after the dose has been introduced into the blister. A foil blister is preferred over capsules as each dose is protected from the ingress of water and penetration of gases such as oxygen in addition to being shielded from light and UV radiation all of which can have a detrimental effect on the delivery characteristics of the inhaler if a dose becomes exposed to them. Therefore, a blister offers excellent environmental protection to each individual drug dose.

Inhalation devices that receive a blister pack comprising a number of blisters each of which contain a pre-metered and individually packaged dose of the drug to be delivered are known. Actuation of the device causes a mechanism to breach or rupture a blister, such as by puncturing it or peeling the lid off, so that when the patient inhales, air is drawn through the blister entraining the dose therein that is then carried out of the blister through the device and via the patient's airway down into the lungs. Pressurized air or gas or other propellants may also be used to carry the dose out of the blister. Alternatively, the mechanism that punctures or opens the blister may push or eject the dose out of the blister into a receptacle from which the dose may subsequently be inhaled.

It is advantageous for the inhaler to be capable of holding a number of doses to enable it to be used repeatedly over a period of time without the requirement to open and/or insert a blister into the device each time it is used. Therefore, many conventional devices include means for storing a number of blisters each containing an individual dose of medicament. When a dose is to be inhaled, an indexing mechanism moves a previously emptied blister away from the opening mechanism so that a fresh one is moved into a position ready to be opened for inhalation of its contents.

An inhaler of the type described above is known from the Applicant's own copending international application no. PCT/GB2004/004416 filed on 18th October 2004 and claiming priority from GB0324358.1 filed 17th October 2003. This international application has been published as W02005/037353 A1.

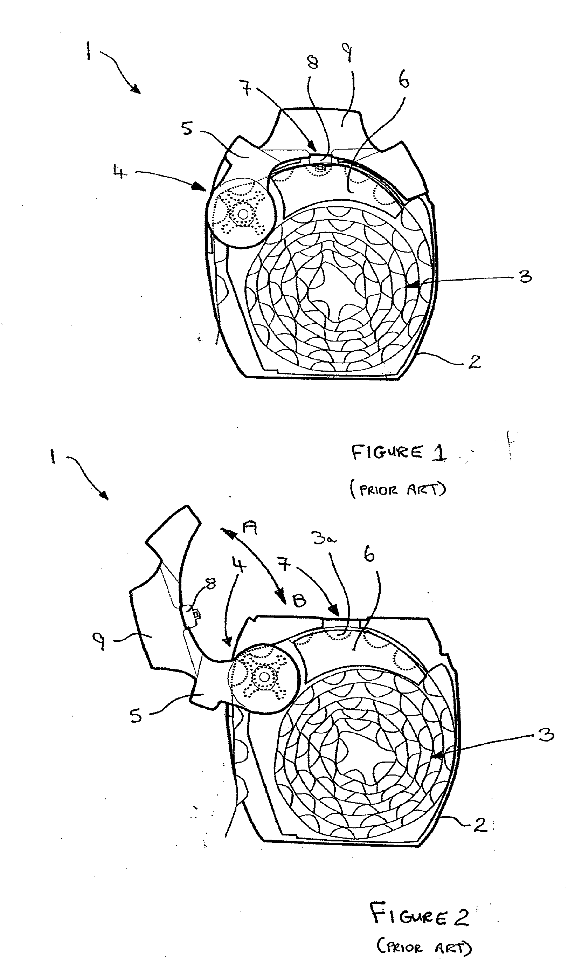

According to one embodiment described and claimed in WO 2005/037353 A1, and illustrated in Figures 1a and 1b of the accompanying drawings, an inhaler 1 has a housing 2 containing a coiled strip of blisters 3. An indexing mechanism 4 comprising a single actuating lever 5 unwinds the coil 3 one blister at a time so that they pass over a blister locator chassis 6 and successively through a blister piercing station 7, when the actuator 5 is pivoted in a direction indicated by arrow "A" in Figure 1b. The blister 3a located at the blister piercing station 7 on each movement of the actuator 5 is pierced on the return stroke of the actuator 5 (in the direction indicated by arrow "B" in Figure 1b) by piercing elements 8 on the actuator 5 itself so that, when a user inhales through a mouthpiece 9, an airflow is generated within the blister 3a to entrain the dose contained therein and carry it out of the blister 3a via the mouthpiece 9 and into the user's airway.

In another embodiment disclosed in WO2005/037353 A1, indexing and piercing of a blister positioned at the blister piercing station 7 is carried out in response to rotation of the cap rather than as a result of direct rotation of the actuator by the user.

The present invention seeks to provide further improved embodiments of inhalation device of the type disclosed in WO2005/03735 A1, that has a relatively simple construction, is robust, straightforward to manufacture and easy for the patient to use.

According to the invention, there is provided an inhaler comprising a housing to receive a strip having a plurality of blisters, each blister having a puncturable lid and containing a dose of medicament for inhalation by a user, a mouthpiece through which a dose of medicament is inhaled by a user, a cap to cover the mouthpiece and, a blister piercing element to pierce the lid of an aligned blister, the cap being rotatable to drive the strip to sequentially move each blister into alignment with the blister piercing element and, an actuator operable in response to rotation of the cap to cause the blister piercing element to puncture the lid of an aligned blister such that, when a user inhales through the mouthpiece, an airflow through the blister is generated to entrain the dose contained therein and carry it out of the blister and via the mouthpiece into the user's airway.

In a preferred embodiment, the actuator is pivotally mounted to the housing such that it rotates in response to rotation of the cap to cause the blister piercing element to pierce the lid of an aligned blister.

In one embodiment, the inhaler preferably comprises a link arm that couples the cap to the actuator to rotate the actuator in response to rotation of the cap.
The link arm may be pivotally mounted to the housing.

The link arm is preferably coupled to the actuator so that the actuator rotates relative to the housing to draw the blister piercing element into the lid of an aligned blister when the link arm rotates.

The inhaler preferably includes cooperating cam elements on the actuator and on the link arm such that rotation of the link arm causes rotation of the actuator to draw the blister piercing element into the lid of an aligned blister.

In one embodiment, the cooperating cam elements comprise a cam follower on the link arm and a cam guide on the actuator, the cam follower and cam guide being configured such that the cam follower follows the cam guide when the link arm rotates to rotate the actuator.

The cam guide may comprise a slot in the actuator and the cam follower may comprise a pin on the link arm slideably received in the slot.

The link arm can be a plate and the actuator may include a leg extending into the housing. In one embodiment, the plate and leg may have a region of overlap so that the pin upstands from the plate and is received in the slot in said region of overlap.

The inhaler preferably comprises a link arm drive element rotatable together with the cap, the drive element and link arm being configured such that they cooperate with each other for part of the rotation of the cap so that the link arm rotates when the user rotates the cap.

The drive element and link arm are preferably configured such that they cooperate when the cap has almost reached its open position so that further rotation of the cap into the open position rotates the link arm.

Advantageously, the drive element and link arm are configured such that they cooperate when the cap has almost reached its closed position so that further rotation of the cap into its closed position rotates the link arm in the opposite direction back to its original position.

The link arm drive element may comprise a first shoulder that engages the link arm to rotate the link arm when the cap is rotated from its closed to its open position and, a second shoulder that engages the link arm to rotate the link arm in the opposite direction when the cap is rotated from its open to its closed position.

In a particular embodiment, the drive element comprises a disc-shaped member.

The drive element can be integral with the cap.

The disc may have an arcuate recess extending around a portion of its circumference and the first and second shoulders can be defined by radially extending walls at each end of the arcuate recess.

In one embodiment, the link arm preferably includes a tooth, the tooth being received within the arcuate recess in the disc such that the first shoulder contacts the tooth as the cap is rotated from its closed to its open position so that further rotation of the cap causes the first shoulder to push against the tooth to rotate the link arm and, such that the second shoulder contacts the tooth as the cap is rotated from its open to its closed position so that further rotation of the cap causes the second shoulder to push against the tooth to rotate the link arm in the opposite direction.

In another embodiment, the cap includes a profiled cam guide and the actuator includes a cam follower that follows the profile of the cam guide during rotation of the cap.

The profiled cam guide may be configured such that, as the cap is rotated, the cam follower follows the profile so as to move the actuator from an initial position into a raised position.

In a most preferred embodiment, the profiled cam guide is configured such that the cam follower follows the profile so as to move the actuator from said initial position into said raised position during rotation of the cap from its open position to its closed position.

In an alternative embodiment, the profiled cam guide may be configured such that the cam follower follows the profile so as to move the actuator from said initial position into said raised position during rotation of the cap from its closed position to its open position.

In yet another alternative embodiment, the profiled cam guide can be configured such that the cam follower follows the profile so as to move the actuator from said initial position into an intermediate position, between the initial position and raised position, during rotation of the cap from its open to its closed positions and, to move the actuator from said intermediate position to said raised position during rotation of the cap from its closed to its open positions. This alternative arrangement has the additional advantage that the space within the cap does not have to be large enough to contain the actuator in its raised position which is necessary when the actuator is raised completely during closure of the cap. On the contrary, the space within the cap only has to be sufficient to contain the actuator in its intermediate position as the raised position is only reached as the cap is opened on a subsequent stroke.

Conveniently, the profiled cam guide can be integral with the cap.

The inhaler may include biasing means associated with the actuator such that the actuator moves from its initial position into its raised position against a biasing force provided by the biasing means that biases the actuator towards its initial position.

In this embodiment, the curved surface ends when the cap reaches its open position and the cam follower falls off the end of the curved surface to release the biasing force and rotate the actuator so that the blister piercing element pierces the lid of an aligned blister.

In one embodiment, the cam guide comprises two cam guide portions and the return path is defined by a channel between the two cam guide portions.

The cam follower may comprise a pin extending from the actuator. The actuator may also comprise a leg extending into the housing with the cam follower upstanding from a free end of the leg.

In a preferred embodiment, the biasing means comprises a spring extending between the actuator and the housing.

In one embodiment, the mouthpiece is formed integrally with the actuator. Alternatively, the actuator is attached to the mouthpiece such that the mouthpiece and actuator rotate together.

In any embodiment of the invention, the mouthpiece and actuator may be intergrated so as to form a mouthpiece/actuator unit, the mouthpiece/actuator unit being operable in response to rotation of the cap to cause the blister piercing element to puncture the lid of an aligned blister such that, when a user inhales through the mouthpiece, an airflow through the blister is generated to entrain the dose contained therein and carry it out of the blister and via the mouthpiece into the user's airway.

Preferably, the mouthpiece/actuator unit comprises an actuator portion and a mouthpiece portion attached to each other. Alternatively, the mouthpiece/actuator unit can be formed or moulded as a single component.

If the mouthpiece portion and actuator portion are separate components, they may be separable from each other.

Advantageously, the mouthpiece/actuator unit includes a flow path for the flow of medicament through the mouthpiece/actuator unit into the patient's airway.

In one embodiment, the blister piercing element comprises an insert, said insert being located within the flow path in the mouthpiece actuator unit, said insert having openings therein for the passage of medicament therethrough. The blister piercing elements conveniently depend from said insert mounted in the flow path.

In other embodiments, the actuator and mouthpiece can be formed as separate components and the mouthpiece can be immovably attached to the housing. In this case, the actuator is mounted for rotation relative to the mouthpiece and to the housing.

When the actuator is mounted for rotation, it may include a pivot arm, a free end of the pivot arm being pivotally mounted to the housing. In one embodiment, the free end of the pivot arm may be captured between the mouthpiece and the housing to pivotally mount the end of the pivot arm.

The mouthpiece preferably includes a flow path for the flow of air and medicament from a punctured blister through the mouthpiece into the patient's airway. A portion of the actuator can be movably received within the flow path in the mouthpiece, said portion having passages therethrough for the flow of air and medicament through said portion. The blister piercing elements can depend from said portion of the actuator mounted in the flow path of the mouthpiece.

In any embodiment of the invention, the inhaler may comprise an indexing mechanism to sequentially move each blister into alignment with the blister piercing element.

In a preferred embodiment, the indexing mechanism comprises a drive member that engages the blister strip to drive the strip to sequentially move each blister into alignment with the blister piercing element.

The cap and drive member are preferably configured such that the cap and drive member cooperate on rotation of the cap from a closed position, in which the cap covers the mouthpiece, towards an open position, in which the mouthpiece is exposed for inhalation through the mouthpiece, so that the drive member drives the strip and moves a blister into alignment with the blister piercing element.

The cap and drive member are preferably configured such that the cap is de-coupled from the drive member as the cap reaches its fully open position, so that there is no drive to the strip as the cap is rotated in the opposite direction from its open position back to its closed position.

In a more preferable embodiment, the cap and drive member are configured such that the cap is de-coupled from the drive member before the cap reaches its open position so that there is no drive to a strip as the cap is rotated further in the same direction towards its open position. In connection with the first embodiment of the invention, this has the advantage that a blister has been aligned with the blister piercing member and movement of the blister strip has stopped prior to piercing of said aligned blister.

It will be appreciated that it is advantageous that the decoupling of the cap and drive member takes place when the cap has reached or has almost reached the open position so that a user can partially open the cap to clean and/or inspect the mouthpiece and close it again without indexing a blister. The return or abort stroke in this case will then cause the strip to move back to its original position as it was prior to movement of the cap, as long as the point at which the cap and drive member become de-coupled has not been reached prior to movement of the cap back towards its closed position.

In one embodiment, the drive member comprises a wheel, the cap and the drive wheel both being mounted to the housing for rotation about the same axis.

According to another aspect, there is provided an inhaler according to the invention containing a strip of blisters each having a puncturable lid and containing a dose of medicament for inhalation by a user.

Embodiments of the invention will now be described, by way of example only, with reference to Figures 3 to 8 of the accompanying drawings, in which:
FIGURE 1 and 2 are side views of a conventional inhalation device to show how a strip is driven to sequentially move blisters into alignment with a blister piercing element by movement of an actuator from the position shown in Figure 1 to the position shown in Figure 2 which drives an indexing wheel. A piercing head on the actuator pierces the lid of an aligned blister when the actuator is returned to its normal position, as shown in Figure 1;
FIGURE 3a is a side view of an inhalation device according to a first embodiment of the present invention with the cap in its closed position covering the mouthpiece;
FIGURE 3b is an enlarged partial view of the inhalation device shown in Figure 3a;
FIGURE 4 is a side view of the inhalation device shown in Figure 1 following rotation of the cap from its closed position to a position just prior to its fully open position;
FIGURE 5 is a side view of the inhalation device shown in Figures 1 and 2 following rotation of the cap from the position shown in Figure 4 to a fully open position;
FIGURE 6a is a side view of an inhalation device according to a second embodiment of the invention with the cap its closed position covering the mouthpiece;
FIGURE 6b is an enlarged partial view of the inhalation device shown in Figure 6a;
FIGURE 7 is a side view of the inhalation device shown in Figure 6 following rotation of the cap from its closed position to its fully open position but before any movement of the actuator has taken place; and
FIGURE 8 is a side view of the inhalation device shown in Figures 6 and 7 after the actuator has dropped to cause the blister piercing member to pierce the lid of an aligned blister.

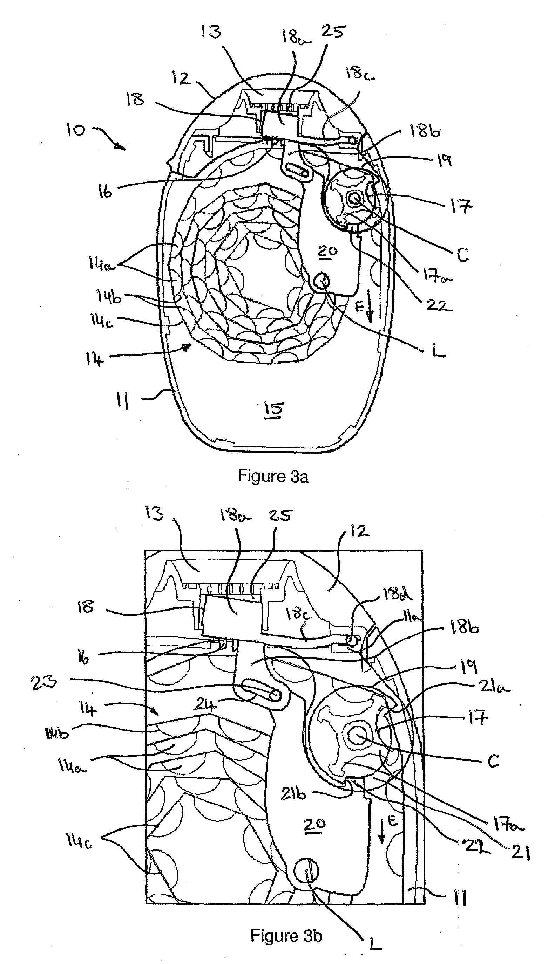

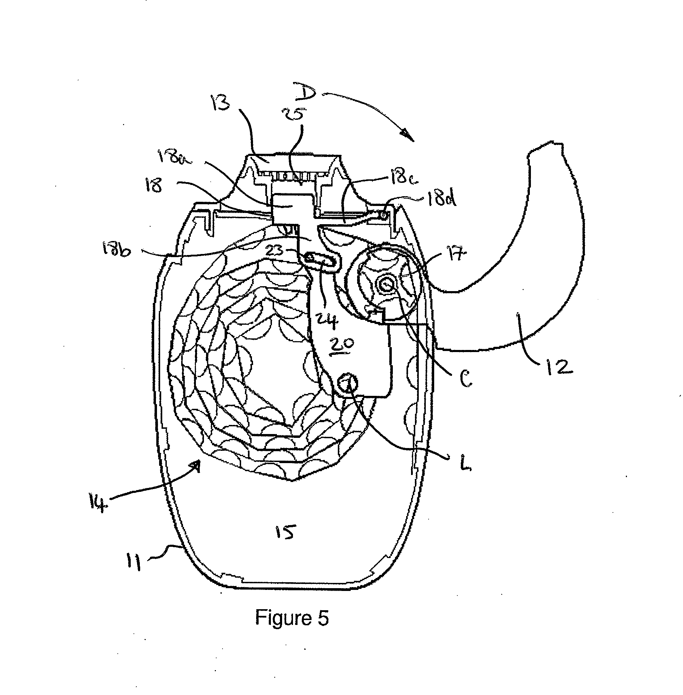

Referring now to the first embodiment illustrated in Figures 3a to 5 of the accompanying drawings, there is shown an inhaler 10 having a housing 11, a cap 12 pivotally mounted to the housing 11 for rotation about an axis marked "C" from a closed position, as shown in Figure 3a and 3b in which the cap 12 covers and protects a mouthpiece 13 to a fully open position, as shown in Figure 5 and indicated by arrow "D", in which the mouthpiece 13 is exposed to enable a user to inhale a dose of medicament through the mouthpiece 13.

For clarity, the inhalation device 10 of the invention is shown with a portion of its housing 11 removed so that its internal workings and components are visible. The components are also visible through the cap 12, although the cap 12 may be opaque in the actual device.

A strip 14 having a plurality of individually spaced moisture proof blisters 14a each containing a pre-measured dose of powdered medicament for inhalation is coiled up within the housing 11. Each blister 14a of the strip 14 comprises a generally hemispherically shaped pocket 14b and a flat puncturable lid 14c permanently heat sealed to the pocket 14b to hermetically seal the dose therein. The strip 14 is preferably manufactured from foil laminate or a combination of foil laminate, such as aluminium, and plastics material.

Although a region 15 is provided within the housing 11 to receive the used portion 14d of the strip 14, it will be appreciated that the invention is also applicable to other inhalation devices (not shown) in which used blisters 14d are not retained within the housing 11 but pass out through an opening (not shown) in the wall of the housing 11 for periodic detachment by a user.

The inhaler 10 includes an indexing mechanism to index the strip 14, i.e. to sequentially move each blister 14b forward by a sufficient distance on each rotation of the cap 12 so as to move a fresh blister 14b into alignment with a blister piercing element 16 which is operable to puncture the lid of an aligned blister 14b to facilitate access to the dose contained therein. Although reference is made to a blister piercing element 16, it will be appreciated that multiple openings are formed in the lid 14c of the blister 14b so that air can be drawn into the blister 14b through one or some of those openings and flow out of the blister 14b, together with an entrained dose of medicament, through one or more other openings.

The indexing mechanism comprises a drive wheel 17 coaxially mounted for rotation along the same axis "C" as the cap. The drive wheel 17 has four spokes 17a. As can be seen in Figure 3a and 3b, the strip 14 passes around the drive wheel 17 and individual blisters 14a are held between the spokes 17a. As the drive wheel 17 rotates (in a clockwise direction as shown in the drawings), the strip 14 is indexed forward, in a direction indicated by arrow "E".

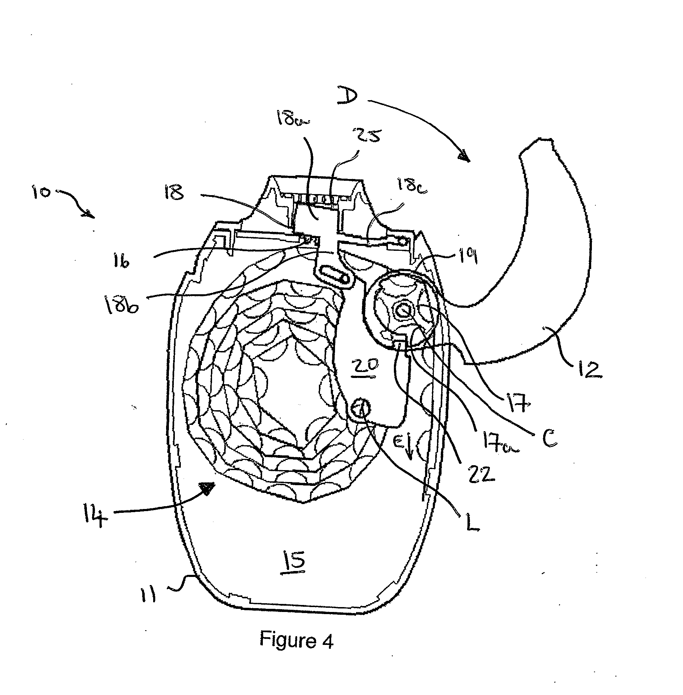

The cap 12 is coupled to the drive wheel 17 by a mechanism (not shown) so that the drive wheel 17 rotates together with the cap 12 as the cap 12 is rotated from its closed position shown in Figure 3, into an intermediate position shown in Figure 4. When the intermediate position of Figure 4 is reached, the next blister 14a is aligned with the blister piercing element 16 and the cap 12 and the drive wheel 17 are de-coupled so that, when the cap 12 is rotated further in the same direction into its fully open position shown in Figure 5, the drive wheel 17 does not rotate together with the cap 12 and so no further indexing or movement of the strip occurs. The last few degrees of movement or overtravel of the cap 12 from its intermediate position shown in Figure 4 to its fully open position shown in Figure 5 is when the blister piercing element 16 is moved (as explained in more detail below) so as to pierce the lid 14c of said aligned blister which has already stopped moving.

The mechanism connecting the drive wheel 17 to the cap 12 may be a clutch or ratchet type mechanism. Mechanisms of this type, i.e. which allow one component to rotate together with another component when said other component is rotated in only one direction are well known. The Applicant's previous application referred to above (WO2005/037353 A1) discloses inhalers having mechanisms of this type for controlling rotation of an indexing wheel in response to rotation of an actuator or cap.

It is important to emphasise that, in a preferred embodiment, the cap 12 and the drive wheel 17 are de-coupled from each other only when the cap 12 reaches the intermediate position and that the intermediate position is only a few degrees short of the fully open position. Therefore, if a user opens the cap 12 partially and then returns it to its closed position without having past the intermediate position, the drive wheel 17 will rotate together with the cap 12 as the cap 12 is rotated back to its closed position, thereby driving the strip backwards (i.e. in the opposite direction to arrow "D" on the drawings) and return the strip 14 to its original position, because the point (i.e. the intermediate position) at which the cap 12 and drive wheel 17 become de-coupled from each other has not been reached. This has the advantage that the user may open the cap 12 until the intermediate position has almost, but not quite, been reached to enable them to inspect and/or clean the mouthpiece 13 and then close it again without having indexed the strip 14 or pierced a blister 14b.

Although piercing of an aligned blister 14b occurs after movement of the strip has stopped, it is envisaged that the mechanism could be configured so that de-coupling of the drive wheel 17 and cap 12 only occurs when the fully open position of the cap 12, as shown in Figure 5, has been reached. In this instance, the blister piercing element 16 will be drawn into and across the lid of a blister as the strip is still being indexed. Alternatively, the blister piercing element 16 may have already pierced and been drawn across the lid of a blister as it is still moving into alignment when the intermediate position shown in Figure 4 has been reached, in which case further movement of the cap 12 from the intermediate position into the fully open position shown in Figure 5 is effectively lost motion, i.e. there is no drive to the drive wheel 17 or coupling to the blister piercing member 16 during these last few degrees of rotation of the cap 12.

The inhaler 10 also comprises an actuator 18. The blister piercing element 16 depends from the actuator 18 and the actuator 18 is rotatable in response to rotation of the cap 12 to cause the blister piercing element 16 to pierce the lid 14c of an aligned blister 14b, as will now be described in more detail.

The inhaler 10 includes a drive element 19 associated with the cap 12 and a link arm 20 rotatably mounted to the housing 11 at point marked "L". The link arm 20 couples the cap 12 to the actuator 18 via the drive element 19 so that, during part of the rotation of the cap 12, the drive element 19 rotates the link arm 20 to cause corresponding rotation of the actuator 18 which draws the blister piercing element 16 into the lid 14c of an aligned blister 14b.

The drive element 19 is rotatable together with the cap 12 and the drive element 19 and cap 12 can be integrally or separately formed, for ease of manufacture, and subsequently attached to each other during assembly. In the illustrated embodiment, the drive element 19 comprises a disc-shaped member having a circumferentially extending recess 21 in its outer surface. The ends of the recess 21 are defined by radially extending walls or shoulders 21 a, 21b that cooperate with the link arm 20 to cause it to rotate.

The link arm 20 comprises a tooth-like protrusion 22 received in the circumferentially extending recess 21 in the disc-shaped member 19. When the cap 12 is rotated from its closed position shown in Figure 3 to an almost fully open or intermediate position shown in Figure 4, there is no interaction between the tooth 22 and disc-shaped member 19. However, in the position shown in Figure 4, the tooth 22 lies in contact with a first shoulder 21 a formed by the recess 21 in the disc-shaped member 19 so that, upon further rotation of the cap 12 from the intermediate position into its fully open position shown in Figure 5, the shoulder 21 a pushes against the tooth 22 to rotate the link arm 20 through a short angle necessary to rotate the actuator 18 and draw the blister piercing element 16 into the lid of an aligned blister 14b.

Although reference is made to the tooth 22 engaging the first shoulder 21a when the cap is almost fully open and, engaging the second shoulder 21b when the cap is almost fully closed, it will be appreciated that the angle or position of the cap 12 between its open and closed positions when the tooth 22 engages with a respective shoulder 21a,21b can be altered by changing the shape of the disc-shaped member, i.e by making the circumferentially extending recess extend over a shorter distance so that the tooth 22 and shoulder 21a,21b engage at a selected position of the cap 12.

The link arm 20 is coupled to the actuator 18 so that, as the link arm 20 rotates, the actuator 18 also rotates to draw the blister piercing member 16 downwardly and into the lid 14c of an aligned blister 14b.

In the illustrated embodiment, the coupling between the link arm 20 and the actuator 18 comprises a cam drive arrangement. A cam follower 23 in the form of a pin upstands from a surface of the link arm 20 and is slideably received in a cam guide slot 24 formed in the actuator 18. The actuator 18 has a body portion 18a and a leg portion 18b that depends downwardly from the body portion 18a into the housing 11, the cam guide slot 24 being formed in the leg 18b. The link arm 20 and leg 18b overlap slightly so that the pin 23 extending from the link arm 20 is received in the cam guide slot 24 in the leg 18b. It will be appreciated that the slot 24 is configured such that, as the link arm 20 rotates, the pin 23 travels along the slot 24 which causes the actuator 18 to rotate and cause the blister piercing element 16 to pierce the lid 14c of an aligned blister, as shown in Figure 5.

The actuator 18 includes a pivot arm 18c extending from the main body 18a and the free end 18d of the pivot arm 18c is pivotally attached to the housing 11. In the illustrated embodiment, the free end 18d of the pivot arm 18c is captured between the mouthpiece 13 and a moulded formation 11a on the housing 11.

When the cap 12 is rotated in the opposite direction from its open to its closed position, the tooth 22 on the link arm 20 again follows the circumferential recess 21 in the disc-shaped member 19 and there is no cooperation between the disc-shaped member 19 and the link arm 20. However, shortly prior to the cap 12 reaching its fully closed position, the tooth 22 contacts the second shoulder 21b at the opposite end of the recess 21 so that, on further rotation of the cap 12 back to its fully closed position, the shoulder 21b and tooth 22 cooperate so as to rotate the link member 20 back into its original position shown in Figure 3. As the link arm 20 rotates, the pin 23 travels back along the cam slot 24 in the actuator 18 causing the actuator 18 to rotate in the opposite direction and lifting the blister piercing element 16 out of the blister 14b.

It will be appreciated that in this embodiment the blister piercing element 16 remains in the blister 14b until the cap 12 is almost completely closed, the link arm 20 and drive element 19 only engaging just prior to the cap 12 reaching its fully closed position. However, it will be appreciated that the inhaler can be easily modified so that the blister piercing element 16 is lifted out of the blister 14b much sooner, i.e. prior to the cap 12 reaching its almost fully closed position. As has already been mentioned above, it is also envisaged that rotation of the actuator 18 and so movement of the blister piercing element 16 into the lid of an aligned blister 14b could occur prior to the cap 12 reaching the almost open position shown in Figure 4, and even when the strip is still being indexed forward in the direction of arrow "E". In this case, the openings formed in the blister lid 14c will be larger than the blister piercing elements 16 as the strip is still being indexed after the piercing elements have punctured the lid 14c and the blister piercing elements 16 will effectively plough down and across the blister lid 14c to open a hole which is larger than the blister piercing element 16, thereby enhancing airflow through the blister.

In the embodiment illustrated in Figures 3 to 5, the actuator 18 and mouthpiece 13 are separate components, the mouthpiece 13 being attached or otherwise immovably fixed to the housing 11. The mouthpiece 13 has a flow path 25 therein for the passage of air and medicament from a punctured blister 14 into the patient's airway and the body portion 18a of the actuator 18 is movably received within the flow path 25 so that the actuator 18 can rotate from the position shown in Figure 4 into the position shown in Figure 5 and back again during rotation of the cap 12. The body portion 18a of the actuator 18 has passages therein (not shown) for the flow of medicament through the actuator 18 into the flow path in the mouthpiece 13. The blister piercing elements 16 depend from the body portion 18a of the actuator 18 in the vicinity of the passages. The blister piercing elements 16 may also bridge the openings to those passages so that the puncture in the blister lid 14c are formed directly beneath those openings.

Although, in the present embodiment, the actuator 18 and the mouthpiece 13 are separate components, it is also envisaged that the actuator may be integrated with the mouthpiece 13 to form a combined mouthpiece/actuator unit which is pivotally mounted to the housing 11. The second embodiment, described below, also takes this configuration.

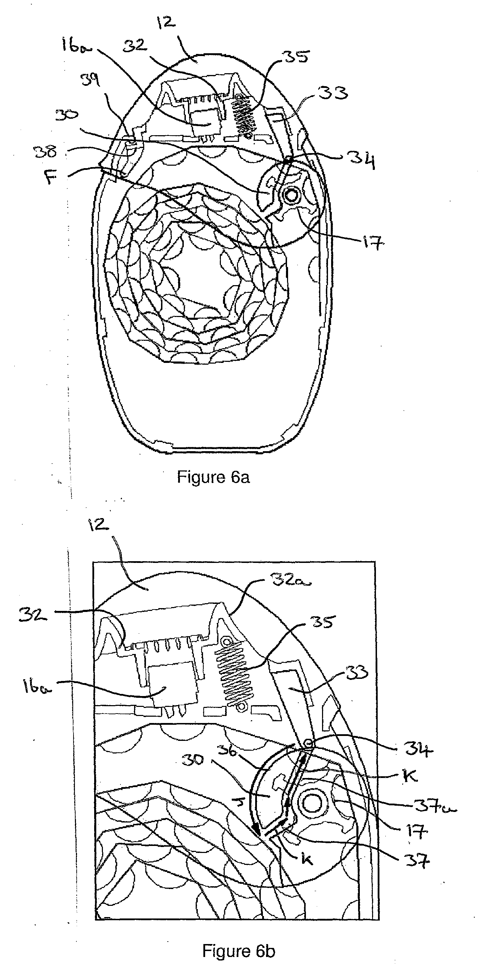

The second embodiment will now be described with reference to Figures 6a to 8 of the accompanying drawings. The indexing mechanism for driving the blister strip 14 is substantially the same as that described with reference to the first embodiment so no further mention of it will be made here. However, the way in which the actuator operates is different and so this will now be described in detail.

In the embodiment of Figures 6a to 8, the drive element comprises a profiled cam guide 30 on the cap 12. As with the previous embodiment, the cam guide 30 can be integrally formed or moulded with the cap 12 or, it can be a separate component which is attached to the cap 12 during manufacture or assembly.

In this embodiment, the actuator and mouthpiece are combined to form a mouthpiece/actuator unit 32 having an upper mouthpiece portion 32a which is shaped so that a user can place it between their lips for inhalation. The combined mouthpiece/actuator unit 32 is mounted to the housing for rotation about axis marked "F" in Figures 6a to 8. The mouthpiece/actuator unit 32 includes an integral arm 33 that extends downwardly from the underside of the mouthpiece/actuator unit into the housing 11 and has a cam follower 34, such as a pin, protruding from its free end (i.e. in a direction perpendicular to the plane of the drawing). The cam follower 34 lies in contact with the cam guide 30 on the cap 12.

A tensioning spring 35 is located within the combined mouthpiece/actuator unit 32 and extends between the mouthpiece 32 and the housing 11. Although a tension coil spring is illustrated in the drawings, the spring may also be a leaf, loop or torsion spring. As explained in more detail below, the spring 35 is preferably in tension when the cap 12 is in its closed position.

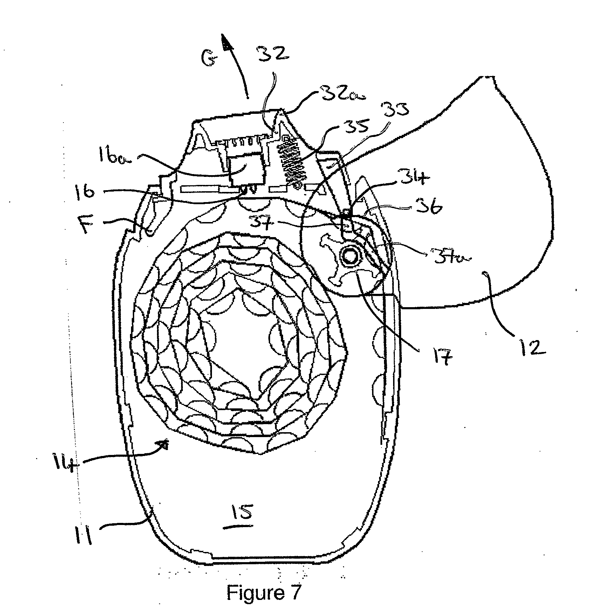

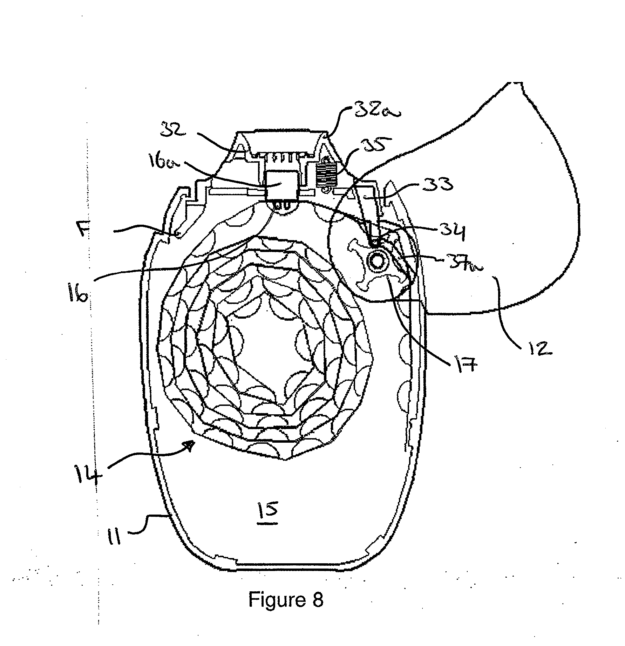

The cam guide 30 has a profiled surface such that, when the cap 12 is rotated from its closed position shown in Figure 6a and 6b to its open position shown in Figures 7 and 8, the cam follower 34 follows the profiled surface of the cam guide 30, the cam follower 34 being held against the cam guide 30 due to the tensioning force provided by the spring 35. More specifically, the cam guide 30 has a curved upper surface 36 so that the cam follower 34 travels along the curved surface 36 when the cap 12 is opened (in the direction of arrow "J" in Figure 6b). In a preferred embodiment, the curved surface 36 is formed such that the follower 34 travels along it during opening of the cap but there is no pivotal movement of the mouthpiece/actuator unit 32 during this time and so no further tensioning of the spring 35. However, in another embodiment, the curved surface 36 may be formed such that there is at least some pivotal movement of the mouthpiece/actuator unit 32 during opening of the cap 12, in the direction of arrow "G" in Figure 7, which causes the spring 35 to be tensioned further i.e. the actuator 32 rotates against a biasing force provided by the spring 35. More specifically, the inhaler may be configured such that the mouthpiece/actuator unit 32 moves into its fully raised position during opening of the cap 12, having already been raised to an intermediate position, so as to partially tension the spring, when the cap 12 was closed on a previous stroke (as described below).

When the cap 12 reaches its fully open position, as shown in Figures 7 and 8, the cam follower 34 clears the end of the curved surface 36 and is free to drop down into a channel 37 (in the direction of arrow "K" in Figure 6b) formed between two portions of the cam guide 30 to rotate the mouthpiece/actuator 32 and cause the blister piercing element 16 to pierce the lid 14c of an aligned blister 14b, as shown in Figure 8. It will be appreciated that the cam follower 34 drops down into the channel 37 under the biasing force stored in the spring 35 when the cam follower 34 clears the end of the curved surface 36. It will be appreciated that when the position shown in Figure 7 is reached, the cam follower 34 will instantaneously drop down the channel 37, as the biasing force of the spring 35 is released, and not remain in the position shown. It is only shown at the top of the channel 37 for ease of understanding.

As the blister piercing element 16 moves so as to pierce the lid 14c of an aligned blister 14b under release of the spring tensioning force provided by the spring 35, a reliable and consistent pierce is achieved and it is not possible to partially pierce a blister 14a. As the piercing element 16 is driven by the release of the tensioning force stored in the spring 35, the piercing velocity/force remains the same each time as it is driven by the spring 35 and is independent of user operation, such as the speed at which the user opens the cap 12.

When the mouthpiece/actuator unit 32 has rotated into its open position shown in Figure 8, the user can now inhale through the mouthpiece/actuator unit 32 to inhale a dose from the blister 14b into their airway. Once inhalation is complete, the user closes the cap 12. When the cap 12 is rotated back towards its closed position, the cam follower 34 follows a return path 37a in the channel 37 (in the direction of arrow "K" in Figure 6b) which preferably lifts the actuator/mouthpiece unit 32 back up into its "home" or initial position shown in Figure 1. During this rotation of the cap 12, tension is generated in the spring 35, keeping the cam follower 34 in contact with the cam guide 30 and loading the spring ready for piercing during the next use. As indicated above, the mouthpiece/actuator unit 32 may be lifted back into its fully raised position during the return stroke of the cap 12 into its closed position so as to fully tension the spring 35. However, it is also envisaged that the mouthpiece/actuator unit 32 may only be partially lifted back towards its raised position and be held in an intermediate position when the cap 12 is fully closed, in which case further lifting of the mouthpiece/actuator unit 32 and tensioning of the spring 35 occurs during the next opening stroke of the cap 12. This arrangement has the further advantage that that the space within the cap 12 does not have to be large enough to contain the mouthpiece/actuator unit 32 in its fully raised position which is necessary when the mouthpiece/actuator unit 32 is raised completely during closure of the cap 12. On the contrary, the space within the cap 12 only has to be sufficient to contain the mouthpiece/actuator unit 32 in its intermediate position as the raised position is only reached when the cap 12 is opened on a subsequent stroke.

In yet another embodiment, there may be no movement of the mouthpiece/actuator unit 32 when the cap 12 is rotated from its open to its closed position. Therefore, there is little or no tension in the spring 35 when the cap 12 is in its closed position. In this case, the mouthpiece/actuator unit 32 is entirely rotated from its initial position to its raised position during opening of the cap 12 to tension the spring 35. This arrangement has the advantage that the spring 35 is not in a loaded state when the inhaler is not in use, thereby enabling a plastic spring to be used. This embodiment also has the advantage that the space within the cap 12 only has to be sufficient to contain the mouthpiece/actuator unit 32 in its initial position and not in an intermediate or raised position.

Although it is envisaged that de-coupling of the cap 12 and drive wheel 17 will take place when the cap 12 reaches its open position, as shown in Figures 7 and 8, it will be appreciated that decoupling of the cap 12 and drive wheel 17 may also occur prior to the fully open position being reached, as with the first embodiment described above, so that there is some movement or overtravel of the cap 12 towards the open position during which there is no rotation of the drive wheel 17.

The housing 11 and mouthpiece/actuator 32 may cooperate when the raised position of the mouthpiece 32 has been reached to prevent any over rotation of the mouthpiece 32. In particular, an edge 38 of the mouthpiece 32 may engage against a shoulder 39 on the housing 11.

The channel 37 may have a reduced depth portion (not shown) at its end close to where the cam follower 34 emerges from the channel 37 when the cap 12 reaches its closed position and the arm 33 may have a degree of flexibility or resilience such that, when the cap 12 approaches its closed position, the cam follower 34 engages the reduced depth portion of the channel 37 so as to flex the arm 33 (in a direction into the page as shown in the drawings), as the cam follower 34 rides up over the reduced depth portion. When the cam follower 34 clears the channel 37, the resilience in the arm 33 causes it to return to its original state. This ensures that the cam follower 34 cannot pass back down the channel 37 when the cap 12 is opened in a subsequent stroke, as it is prevented from doing so as the cam follower 34 is blocked by the reduced depth portion of the channel 37. Preferably, there is a gradual reduction in depth extending along at least a part of the channel 37 so that the cam follower 34 travels up onto the reduced depth portion and gradually flexes the arm 33.

In this second embodiment, the blister piercing element 16 may include an insert 16a that is clipped or otherwise immovably fixed within a flow path channel of the mouthpiece/actuator unit 32. The insert 16a may be removable from the flow path channel for cleaning and/or replacement.

Although the second embodiment has a combined actuator/mouthpiece unit 32, it is also envisaged that the mouthpiece 32 could be fixed to the housing 11, as in the first embodiment described with reference to Figures 3 to 5. In this case, the actuator would be a separate component, similar to the actuator 18 of Figures 3 to 5, that is pivotally mounted with respect to the housing and to the mouthpiece.

Many modifications and variations of the invention falling within the terms of the following claims will be apparent to those skilled in the art and the foregoing description should be regarded as a description of the preferred embodiments of the invention only. For example, although reference is made to a "mouthpiece", the invention is also applicable to devices in which the dose is inhaled through the nasal passages. Therefore, for the purposes of this specification, the term "mouthpiece" should also be construed so as to include within its scope a tube which is inserted into the nasal passages of a patient for inhalation therethrough.

It will be appreciated that the inhalation device of the present invention may be used in conjunction with a spiral wound element and/or a fixed or flexible wall separating a chamber containing unused blisters from a chamber that receives the used blisters. Such modifications are known from the Applicant's own earlier European patent applications nos. 07111998.6 and 07111996.0.

## Claims

1. An inhaler comprising a housing to receive a strip having a plurality of blisters, each blister having a puncturable lid and containing a dose of medicament for inhalation by a user, a mouthpiece through which a dose of medicament is inhaled by a user, a cap to cover the mouthpiece and, a blister piercing element to pierce the lid of a blister, the cap being rotatable to drive the strip to sequentially move each blister into alignment with the blister piercing element and, an actuator operable in response to rotation of the cap to cause the blister piercing element to puncture the lid of an aligned blister such that, when a user inhales through the mouthpiece, an airflow through the blister is generated to entrain the dose contained therein and carry it out of the blister and via the mouthpiece into the user's airway.

2. An inhaler according to claim 1, wherein the actuator is pivotally mounted to the housing such that it rotates in response to rotation of the cap to cause the blister piercing element to pierce the lid of an aligned blister.

3. An inhaler according to claim 2, comprising a link arm that couples the cap to the actuator to rotate the actuator in response to rotation of the cap.

4. An inhaler according to claim 3, wherein the link arm is pivotally mounted to the housing.

5. An inhaler according to claim 4, wherein the link arm is coupled to the actuator so that the actuator rotates relative to the housing to draw the blister piercing element into the lid of an aligned blister when the link arm rotates.

6. An inhaler according to claim 5, including cooperating cam elements on the actuator and on the link arm such that rotation of the link arm causes rotation of the actuator to draw the blister piercing element into the lid of an aligned blister.

7. An inhaler according to claim 6, wherein the cooperating cam elements comprise a cam follower on the link arm and a cam guide on the actuator, the cam follower and cam guide being configured such that the cam follower follows the cam guide when the link arm rotates to rotate the actuator.

8. An inhaler according to claim 7, wherein the cam guide comprises a slot in the actuator and the cam follower comprises a pin on the link arm slideably received in the slot.

9. An inhaler according to claim 8, wherein the link arm comprises a plate and the actuator includes a leg extending into the housing, the plate and leg having a region of overlap, the pin upstanding from the plate and being received in the slot in said region of overlap.

10. An inhaler according to any preceding claim, comprising a link arm drive element rotatable together with the cap, the drive element and link arm being configured such that they cooperate with each other for part of the rotation of the cap so that the link arm rotates when the user rotates the cap.

11. An inhaler according to claim 10, wherein the drive element and link arm are configured such that they cooperate when the cap has almost reached its open position so that further rotation of the cap into the open position rotates the link arm.

12. An inhaler according to claim 11, wherein the drive element and link arm are configured such that they cooperate when the cap has almost reached its closed position so that further rotation of the cap into its closed position rotates the link arm in the opposite direction back to its original position.

13. An inhaler according to any of claims 10 to 12, wherein the link arm drive element comprises a first shoulder that engages the link arm to rotate the link arm when the cap is rotated from its closed to its open position and, a second shoulder that engages the link arm to rotate the link arm in the opposite direction when the cap is rotated from its open to its closed position.

14. An inhaler according to claim 13, wherein the drive element comprises a disc-shaped member.

15. An inhaler according to any of claims 10 to 14, wherein the drive element is integral with the cap.

16. An inhaler according to claim 14 or 15, wherein the disc has an arcuate recess extending around a portion of its circumference and the first and second shoulders are defined by radially extending walls at each end of the arcuate recess.

17. An inhaler according to claim 16, wherein the link arm includes a tooth, the tooth being received within the arcuate recess in the disc such that the first shoulder contacts the tooth as the cap is rotated from its closed to its open position so that further rotation of the cap causes the first shoulder to push against the tooth to rotate the link arm and, such that the second shoulder contacts the tooth as the cap is rotated from its open to its closed position so that further rotation of the cap causes the second shoulder to push against the tooth to rotate the link arm in the opposite direction.

18. An inhaler according to claim 1 or claim 2, wherein the cap includes a profiled cam guide and the actuator includes a cam follower that follows the profile of the cam guide during rotation of the cap.

19. An inhaler according to claim 18, wherein the profiled cam guide is configured such that, as the cap is rotated the cam follower follows the profile so as to move the actuator from an initial position into a raised position.

20. An inhaler according to claim 19, wherein the profiled cam guide is configured such that the cam follower follows the profile so as to move the actuator from said initial position into said raised position during rotation of the cap from its open to its closed positions.

21. An inhaler according to claim 19, wherein the profiled cam guide is configured such that the cam follower follows the profile so as to move the actuator from said initial position into said raised position during rotation of the cap from its closed to its open positions.

22. An inhaler according to claim 19, wherein the profiled cam guide is configured such that the cam follower follows the profile so as to move the actuator from said initial position into an intermediate position during rotation of the cap from its open to its closed positions and, to move the actuator from said intermediate position to said raised position during rotation of the cap from its closed to its open positions.

23. An inhaler according to any of claims 19 to 22, wherein the profiled cam guide is integral with the cap.

24. An inhaler according to any of claims 19 to 23, including biasing means associated with the actuator such that the actuator moves from its initial position into its raised position against a biasing force provided by the biasing means that biases the actuator towards its initial position.

25. An inhaler according to claim24, wherein the cam guide has a curved surface and the cam follower follows the curved surface as the cap is rotated from its closed into its open position.

26. An inhaler according to claim 25, wherein the curved surface ends when the cap reaches its open position, the cam follower falling off the end of the curved surface to release the biasing force and rotate the actuator so that the blister piercing element pierces the lid of an aligned blister.

27. An inhaler according to claim 26, wherein the cam guide has a return path and the cam follower follows the return path as the cap is rotated back from its open to its closed position.

28. An inhaler according to claim 27, wherein the cam guide comprises two cam guide portions and the return path is defined by a channel between the two cam guide portions.

29. An inhaler according to any of claims 18 to 28, wherein the cam follower comprises a pin extending from the actuator.

30. An inhaler according to any of claims 18 to 29, wherein the actuator comprises a leg extending into the housing and the cam follower upstands from a free end of the leg.

31. An inhaler according to any of claims 24 to 30, wherein the biasing means comprises a spring extending between the actuator and the housing.

32. An inhaler according to any preceding claim, wherein the mouthpiece and actuator are integrated so as to form a mouthpiece/actuator unit, the mouthpiece/actuator unit being operable in response to rotation of the cap to cause the blister piercing element to puncture the lid of an aligned blister such that, when a user inhales through the mouthpiece, an airflow through the blister is generated to entrain the dose contained therein and carry it out of the blister and via the mouthpiece into the user's airway.

33. An inhaler according to claim 32, wherein the mouthpiece/actuator unit comprises an actuator portion and a mouthpiece portion attached to each other.

34. An inhaler according to claim 33, wherein the actuator portion and the mouthpiece portion are separable from each other.

35. An inhaler according to any of claims 32 to 34, wherein the mouthpiece/actuator unit includes a flow path for the flow of medicament through the mouthpiece/actuator unit into the patient's airway.

36. An inhaler according to claim 35, wherein the blister piercing element comprises an insert, said insert being located within the flow path in the mouthpiece actuator unit, said insert having openings therein for the passage of medicament therethrough.

37. An inhaler according to claim 36, wherein the blister piercing elements depend from said insert mounted in the flow path.

38. An inhaler according to any of claims 1 to 31, wherein the actuator and mouthpiece are separate components, the mouthpiece being attached to the housing and the actuator being mounted for rotation relative to the mouthpiece and to the housing.

39. An inhaler according to claim 38, wherein the actuator includes a pivot arm, a free end of the pivot arm being pivotally mounted to the housing.

40. An inhaler according to claim 39, wherein the free end of the pivot arm is captured between the mouthpiece and the housing to pivotally mount the end of the pivot arm.

41. An inhaler according to any of claims 38 to 40, wherein the mouthpiece includes a flow path for the flow of air and medicament from a punctured blister through the mouthpiece into the patient's airway, a portion of the actuator being movably received within with the flow path in the mouthpiece, said portion having passages therethrough for the flow of air and medicament through said portion.

42. An inhaler according to claim 41, wherein the blister piercing elements depend from said portion of the actuator mounted in the flow path of the mouthpiece.

43. An inhaler according to any preceding claim, comprising an indexing mechanism to sequentially move each blister into alignment with the blister piercing element.

44. An inhaler according to claim 43, wherein the indexing mechanism comprises a drive member that engages the blister strip to drive the strip to sequentially move each blister into alignment with the blister piercing element.

45. An inhaler according to claim 44, wherein the cap and drive member are configured such that the cap and drive member cooperate on rotation of the cap from a closed position, in which the cap covers the mouthpiece, to an open position, in which the mouthpiece is exposed for inhalation through the mouthpiece, so that the drive member drives the strip and moves a blister into alignment with the blister piercing element.

46. An inhaler according to claim 45, wherein the cap and drive member are configured such that the cap is de-coupled from the drive member as the cap reaches its fully open position, so that there is no drive to a strip as the cap is rotated in the opposite direction from its open position back to its closed position.

47. An inhaler according to claim 45, wherein the cap and drive member are configured such that the cap is de-coupled from the drive member before the cap reaches its open position so that there is no drive to a strip as the cap is rotated further in the same direction towards its open position.

48. An inhaler according to any of claims 44 to 47, wherein the drive member comprises a wheel, the cap and the drive wheel both being mounted to the housing for rotation about the same axis.

49. An inhaler according to any preceding claim containing a strip of blisters each having a puncturable lid and containing a dose of medicament for inhalation by a user.
